Europäisches Patentamt

European Patent Office

Office européen des brevets

(19)

(11) Numéro de publication: **0 088 694
B1**

## FASCICULE DE BREVET EUROPEEN

(12)

(45) Date de publication du fascicule du brevet:
25.06.86

(21) Numéro de dépôt: 83400459.0

(22) Date de dépôt: 07.03.83

(51) Int. Cl.⁴: **A 61 K 39/395**

(54) **Médicament cytotoxique formé de l'association d'au moins une immunotoxine et de la chloroquine.**

(30) Priorité: **10.03.82 FR 8204047**

(43) Date de publication de la demande:
**14.09.83 Bulletin 83/37**

(45) Mention de la délivrance du brevet:
**25.06.86 Bulletin 86/26**

(84) Etats contractants désignés:
**AT BE CH DE FR GB IT LI LU NL SE**

(56) Documents cités:
**FR - A - 2 466 252**

**BIOLOGICAL ABSTRACTS, vol. 73, pages 33-38, no.
78102, Philadelphia Pennsylvania USA; Y.J. SCHNEIDER
et al.: "Effect of chloriquine and methylamine on
endocytosis of fluorescein-labeled control
immunoglobulin G and of anti-plasma membrane
immunoglobulin G by cultured fibroblasts"
CHEMICAL ABSTRACTS, vol. 92, no. 25, 23 juin 1980,
page 141, no. 209920u, Columbus Ohio USA; S.H.
LEPPLA et al.: "Inhibition of diphteria toxin degradation
and cytotoxic action by chloroquine".**

(73) Titulaire: **SANOFI, Société dite:, 40, Avenue George V,
F-75008 Paris (FR)**

(72) Inventeur: **Jansen, Franz, Chemin des Sesquets,
F-34160 Assas (FR)**
Inventeur: **Gros, Pierre, 18 rue des Muriers,
F-34100 Montpellier (FR)**

(74) Mandataire: **Gillard, Marie-Louise et al, Cabinet Beau de
Loménie 55, Rue d'Amsterdam, F-75008 Paris (FR)**

## Description

La présente invention concerne les médicaments comportant, en association, au moins une immunotoxine et de la chloroquine.

Dans les demandes de brevets français antérieurs, portant notamment les numéros 7 827 838 (FR-A 2 437 213), 7 924 655 (FR-A 2 466 252), 8 107 596 (EP-A 63 988) et 8 121 836 (EP-A 80 401), on a décrit la préparation de produits anticancéreux dits conjugués obtenus par couplage, par liaison covalente, de la chaîne A de la ricine avec des anticorps ou fragments d'anticorps dirigés contre un antigène porté par une cellule à détruire. Les produits de ce type sont désignés dans la présente demande sous le nom générique d'immuntoxines.

Dans la demande européenne EP-A 80 401 on a décrit en outre la propriété des ions ammonium (sous la forme d'un quelconque de leurs sels et en particulier le chlorure) de potentialiser de façon efficace l'action cytotoxique de ces immunotoxines.

Dans une autre demande antérieure déposée en France sous le n° 82 02 091 (EP-A 86152) la demanderesse a décrit la propriété des substances appartenant à la classe des ionophores carboxyliques de potentialiser l'activité des immunotoxines et d'accélérer leur cinétique d'action, selon des modalités similairs à celles déjà décrites pour les ions ammonium.

Toutes ces substances potentialisatrices et accélératrices peuvent être utilisées pour améliorer l'efficacité des immunotoxines, soit in vitro, soit in vivo chez l'homme ou l'animal, dans un but thérapeutique.

La présente invention a pour objet la préparation de médicaments cytotoxiques puissants utilisant la potentialisation des effets cytotoxiques sélectifs des immunotoxines décrites dans les demandes de brevet ci-dessus mentionnées.

Après que de nombreuses autres substances ont été étudiées sans succès, il a été trouvé que la chloroquine utilisée sous la forme de l'un quelconque de ses sels pharmaceutiquement acceptables était une substance particulièrement intéressante pour potentialiser et accélérer l'effet cytotoxique sélectif des immunotoxines.

En effet, et en particulier pour les applications thérapeutiques in vivo, la chloroquine possède l'avantage d'avoir été utilisée comme médicament chez l'homme depuis de nombreuses années. Ses modalités d'utilisation en thérapeutique sont donc très bien connues. C'est un médicament bien toléré jusqu'à des doses relativement élevées (de l'ordre de 2 g en une seule prise chez l'homme adulte) et qui présente des caractéristiques pharmacocinétiques particulièrement intéressantes dues à une élimination urinaire lente, pour 70% de la dose sous forme inchangée et à une demi-vie sérique très longue (40 à 70 heures).

Dans la présente invention, il a été trouvé que, de façon surprenante, la chloroquine utilisée à des doses où elle ne présente elle-même aucune cytotoxicité propre pour les lignées étudiées, potentialise de façon extrêmement importante (facteur de 2500) la cytotoxicité spécifique des immunotoxines.

L'exemple suivant non limitatif permet de mieux comprendre la portée de l'invention.

Exemple

Cet exemple démontre la potentialisation de la cytoxicité sélective de l'immunotoxine anti-T65 (telle que décrite dans la demande EP-A 80 401 de la demanderesse) vis-à-vis des cellules lymphoblastoïdes T humaines de la lignée CEM portant l'antigène T65.

Dans ces expériences, la cytotoxicité a été évaluée par la mesure de l'incorporation de $^{14}$C-leucine par les cellules après 18 h d'incubation à 37°C en présence de quantités connues de l'immunotoxine étudiée, ou de substances cytotoxiques de références, en absence ou en présence de chloroquine.

1. Potentialisation de l'effet cytotoxique par la chloroquine.

Les résultats de ces expériences sont présentés sous la forme de courbes dose/effet présentant en ordonnée l'effet cytotoxique évalué comme indiqué ci-dessus par l'incorporation du traceur, calculée en % de la valeur obtenue sur les cellules témoins sans substance cytotoxique et en abscisse les concentrations molaires en sous-unité toxique des substances cytotoxiques étudiées. La chloroquine a été testée à la concentration de 60 micromolaires. Il a été préalablement vérifié que la chloroquine n'est pas spontanément cytotoxique pour les cellules employées, à la concentration indiquée.

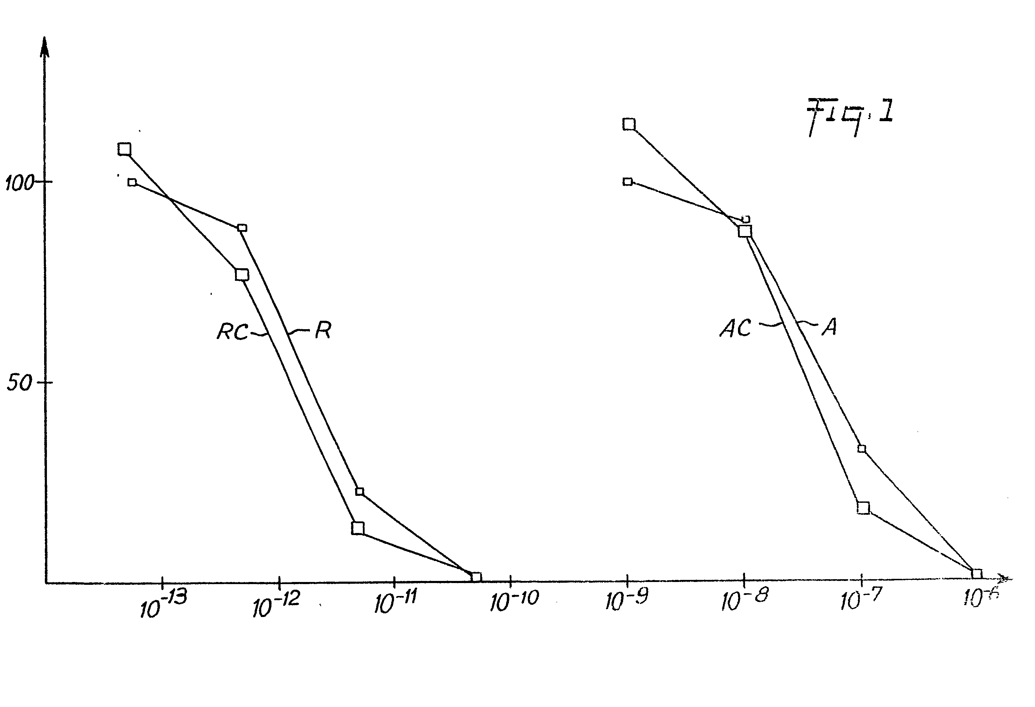

La figure 1 montre l'effet de la chloroquine sur la cytotoxicité propre de la ricine et de sa chaîne A isolée, prises comme substances de références. Les valeurs des concentrations molaires (CI50) correspondant à 50% d'inhibition d'incorporation du traceur sont indiquées dans le tableau I.

Sur cette figure 1, ont été reportés respectivement les résultats obtenus pour la ricine (R), le mélange ricine et chloroquine (R-C) la chaîne A de la ricine (A) et le mélange de la chaîne A de la ricine avec la chloroquine (A-C).

Tableau I

| Substances testées | Avec chloroquine 60 $\mu$M | Sans chloroquine |
|---|---|---|
| ricine | 1,2.10$^{-12}$ M | 1,9.10$^{-12}$ M |
| chaîne A | 3,5.10$^{-8}$ M | 5.10$^{-8}$ M |

Ces résultats démontrent qu'il n'y a pratiquement pas d'effet potentialisateur de la chloroquine sur la cytotoxicité de la ricine (facteur de potentialisation de 1,6) et de la chaîne A (facteur de potentialisation de 1,4).

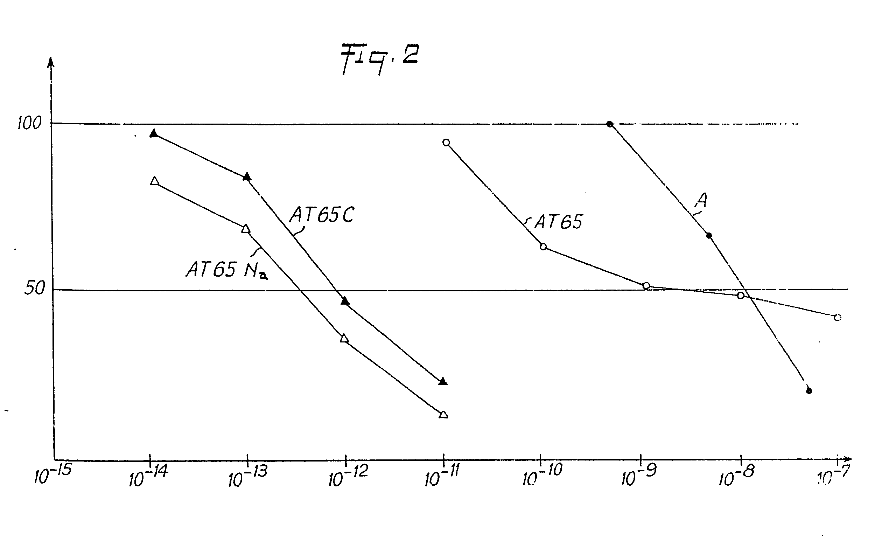

La figure 2 montre l'effet potentialisateur comparé de l'ion NH$_4$+ (10 mM) et de la chloroquine

(60 μM) sur la cytotoxicité de l'immunotoxine anti-T65 vis-à-vis des cellules de la lignée CEM. Les valeurs des concentrations molaires (CI50) correspondant à 50% d'inhibition d'incorporation du traceur sont rappelées dans le tableau II.

Sur cette figure 2, ont été reportés respectivement les résultats obtenus avec le conjugué anti-T65 (AT65), la chaîne A de la ricine (A), et les mélanges de conjugué anti-65 avec, d'une part, un sel d'ammonium quaternaire (AT65 Na) et, d'autre part, la chloroquine (AT65 C).

Ces résultats montrent que l'effet potentialisateur de la chloroquine sur l'activité de l'immunotoxine vis-à-vis de sa cible spécifique est voisin de celui de l'ion ammonium et de l'ordre d'un facteur de 2500. Ceci signifie qu'en présence de chloroquine l'immunotoxine anti-T65 est vis-à-vis de sa cible spécifique un agent cytoxique plus puissant que la ricine elle même.

En outre, la chloroquine présente la propriété remarquable non seulement de potentialiser l'activité mais aussi d'augmenter la sélectivité de l'immunotoxine. Si l'on prend en effet comme critère de sélectivité d'action de l'immunotoxine le rapport des CI50 de la chaîne A libre et de l'immunotoxine, ce rapport est de 10 en l'absence de chloroquine et voisin de 44 000 en présence de chloroquine.

Tableau II

| Substances potentialisatrices testées | CI50 |
|---|---|
| aucune | $5.10^{-9}$ M |
| NH$_4$$^+$10 mM | $3.10^{-13}$ M |
| chloroquine 60 μM | $8.10^{-13}$ M |

2. Accélération des cinétiques de cytoxicité par la chloroquine.

L'effet de la chloroquine ne se limite pas à accroître d'une façon considérable l'activité cytotoxique et la sélectivité des immunotoxines. Cette substance permet aussi d'accélérer de façon très importante la cinétique de cytoxicité des immunotoxines, comme le montre l'expérience suivante:

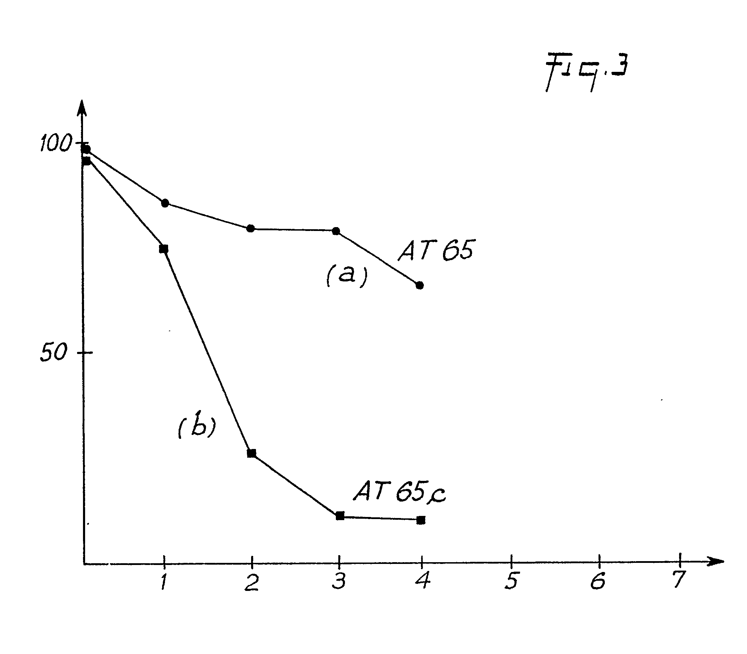

Dans cette expérience, on a mesuré comme précédemment l'incorporation de traceur radioactif dans les cellules mais cette fois en fonctions du temps d'incubation des cellules avec l'immunotoxine, en absence et en présence de chloroquine 60 μM comme potentialisateur. Cette expérience a été réalisée sur le modèle cellulaire constitué par la lignée lymphoblastoïde humaine CEM avec l'immunotoxine anti-T65 à la concentration de 50 nM. Les résultats sont présentés dans la figure 3.

Sur cette figure, ont été représentés en fonction du temps (heures) les résultats obtenus avec le conjugué anti-T65 (AT65) et avec le mélange du conjugué anti-T65 avec la chloroquine (AT65 C).

Pour cette lignée, il apparaît qu'en l'absence de potentialisation la cinétique de cytoxicité est très lente comme le montre la courbe a. D'autres expériences dans les mêmes conditions ont montré que le temps nécessaire à obtenir 50% de réduction de l'incorporation du traceur était de l'ordre de 20 h. Par contre, en présence de chloroquine, une accélération spectaculaire de la cinétique se manifeste (courbe b) puisque le temps nécessaire à obtenir 50% d'inhibition d'incorporation est alors de l'ordre de 1,5 h seulement.

Un tel effet d'accélération est de la plus haute importance pour toutes applications des immunotoxines et en particulier pour les applications thérapeutiques in vivo car la rapidité d'action du médicament est toujours un facteur très favorable à l'efficacité du traitement.

On peut donc utiliser en tant que médicament en thérapeutique humaine l'association constituée par immunotoxin et la chloroquine (sous la forme de la base ou d'un quelconque de ses sels pharmaceutiquement acceptables). Il peut être utilisé pour le traitement des affections, cancéreuses ou non, qui seraient sensibles à l'anticorps utilisé pour la préparation de l'immunotoxine.

Visant à éliminer la totalité des cellules cancéreuses, le traitement devra être effectué avec une dose suffisante d'immunotoxine associée avec une quantité de chloroquine pouvant varier de 10 mg à 2 g (exprimés en base) à chaque administration d'immunotoxine. La durée du traitement devra être déterminée dans chaque cas en fonctions su sujet et de la nature de l'affection à traiter.

Les nouveaux médicaments selon l'invention sont conditionnés pour pouvoir être utilisés dans les conditions adaptées à leur emploi. L'immunotoxine sera administrée par voie injectable et de préférance par voie intraveineuse. la chloroquine sera de préférence administrée par voie injectable sauf si son emploi par voie orale présentait des avantages thérapeutiques.

**Revendications pour les états contractants BE, CH, DE, FR, GB, IT, LI, LU, NL, SE**

1. Médicaments cytotoxiques caractérisés en ce qu'ils comportent, en association, au moins une immunotoxine obtenue par couplage, par liaison covalente, de la chaîne A de la ricine avec des anticorps ou fragments d'anticorps dirigés contre un antigène porté par une cellule à détruire et de la chloroquine ou un sel pharmaceutiquement acceptable de la chloroquine.

2. Utilisation de la chloroquine ou d'un de ses sels pharmaceutiquement acceptables pour la potentialisation de l'effet cytotoxique des immunotoxines obtenues par couplage, par liaison covalente, de la chaîne A de la ricine avec des anticorps ou fragments d'anticorps dirigés contre un antigène porté par une cellule à détruire.

**Revendication pour l'état contractant AT**

Utilisation de la chloroquine ou d'un de ses sels pharmaceutiquement acceptables pour la potentialisation de l'effet cytotoxique des immunotoxines obtenues par couplage, par liaison covalente, de la chaîne A de la ricine avec des anticorps ou fragments d'anticorps dirigés contre un antigène porté par une cellule à détruire.

## Patentansprüche für die Vertragsstaaten BE, CH, DE, FR, GB, IT, LI, LU, NL, SE

1. Zytotoxische Medikamente, dadurch gekennzeichnet, dass sie mindestens ein Antitoxin, das durch Koppelung der Kette A des Ricins mit Antikörpern oder Antikörperfragmenten, die gegen ein von einer zu vernichtenden Zelle getragenes Antigen gerichtet sind, mittels kovalenter Bindung erhalten ist, in Verbindung mit Chloroquin oder einem pharmazeutisch akzeptablen Salz von Chloroquin enthalten.

2. Verwendung von Chloroquin oder eines seiner pharmazeutisch akzeptablen Salze zur Potenzierung der zytotoxischen Wirkung von Antitoxinen, die durch Koppelung der Kette A des Ricins mit Antikörpern oder Antikörperfragmenten, die gegen ein von einer zu vernichtenden Zelle getragenes Antigen gerichtet sind, mittels kovalenter Bindung erhalten sind.

## Patentanspruch für den Vertragsstaat AT

Verwendung von Chloroquin oder eines seiner pharmazeutisch akzeptablen Salze zur Potenzierung der zytotoxischen Wirkung von Antitoxinen, die durch Koppelung der Kette A des Ricins mit Antikörpern oder Antikörperfragmenten, die gegen ein von einer zu vernichtenden Zelle getragenes Antigen gerichtet sind, mittels kovalenter Bindung erhalten sind.

## Claims for the contracting states: BE, CH, DE, FR, GB, IT, LI, LU, NL, SE

1. Cytotoxic drugs characterized in that they comprise, in association, at least one immunotoxin obtained by coupling, by covalent bond, of the A chain of ricin with antibodies or antibody fragments directed against an antigen carried by a cell to be destroyed, and with chloroquine or a pharmaceutically acceptable salt thereof.

2. Use of chloroquine or one of its pharmaceutically acceptable salts for potentiating the cytotoxic effects of the immunotoxins obtained by coupling, by covalent bond, of the A chain of ricin with antibodies or antibody fragments directed against an antigen carried by a cell to be destroyed.

## Claim for the contracting state: AT

Use of chloroquine or of one of its pharmaceutically acceptable salts for potentiating the cytotoxic effects of immunotoxins obtained by coupling, by covalent bond, of the A chain of ricin with antibodies or antibody fragments directed against an antigen carried by a cell to be destroyed.

Fig. 1

Fig. 2

Fig. 3